# EUROPÄISCHE PATENTSCHRIFT

(11) **EP 1 585 730 B1**
(45) Veröffentlichungstag und Bekanntmachung des Hinweises auf die Patenterteilung: **09.03.2011**
(21) Anmeldenummer: 04700684.6
(22) Anmeldetag: 08.01.2004
(51) Int. Cl.: C07D 213/64, C07D 241/18, C07D 265/32, C07D 265/06, C07D 265/10, C07F 9/09, A61K 31/4412, A61K 31/5375, A61K 31/4965, A61P 7/00, A61P 9/00

(54) **CARBONSÄUREAMIDDERIVATE UND IHRE VERWENDUNG ALS FAKTOR XA INHIBITOREN**
CARBOXAMIDE DERIVATIVES AND THEIR USE AS FACTOR XA INHIBITORS
DERIVES D'AMIDE D'ACIDE CARBOXYLIQUE ET UTILISATION DE CES COMPOSES EN TANT QU'INHIBITEURS DU FACTEUR XA

(30) Priorität: 23.01.2003 DE 10302500
(43) Veröffentlichungstag der Anmeldung: 19.10.2005
(73) Patentinhaber: Merck Patent GmbH, 64293 Darmstadt (DE)
(72) Erfinder: DORSCH, Dieter, 64372 Ober-Ramstadt (DE); CEZANNE, Bertram, 64546 Mörfelden-Walldorf (DE); MEDERSKI, Werner, 64673 Zwingenberg (DE); TSAKLAKIDIS, Christos, 69469 Weinheim (DE); GLEITZ, Johannes, 64289 Darmstadt (DE); VAN AMSTERDAM, Christoph, 64295 Darmstadt (DE)
(86) Internationale Anmeldenummer: PCT/EP2004/000061
(87) Internationale Veröffentlichungsnummer: WO 2004/065369

(56) Entgegenhaltungen:
- WO-A-02/48099
- WO-A-02/074735
- WO-A-02/083630
- WO-A-2004/031145

## Beschreibung

Die Erfindung betrifft 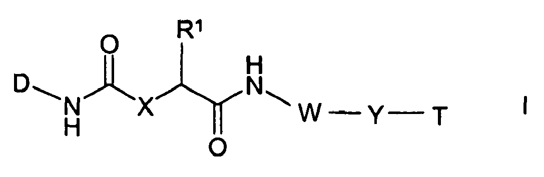 worin
- D: einfach durch Hal substituiertes Phenyl,
- R¹: Alkyl mit 1, 2, 3, 4, 5 oder 6 C-Atomen, das einfach durch S(O)ₘR², SO₂N(R²)₂, SO₃R², S(=O)(=NR²)R², NR²SO₂R², OSO₂R², OSO₂N(R²)₂ oder PO(OR²)₂ substituiert ist,
- R²: H oder Alkyl mit 1, 2, 3, 4, 5 oder 6 C-Atomen,
- W: -(CH₂)ₙ-,
- X: NH oder O,
- Y: Ar-diyl,
- T: Piperidin-1-yl, 2-Oxo-piperidin-1-yl, 2-Oxo-pyrrolidin-1-yl, Pyrro- lidin-1-yl, 3-Oxo-morpholin-4-yl, Morpholin-4-yl, 4-Oxo-1*H*- pyridin-1-yl, 2,6-Dioxo-piperidin1-yl, 2-Oxo-piperazin-1-yl, 2,6- Dioxo-piperazin-1-yl, 2,5-Dioxo-pyrrolidin-1-yl, 2-Oxo-1,3- oxazolidin-3-yl, 3-Oxo-2*H*-pyridazin-2-yl, 2-Caprolactam-1-yl (= 2-Oxo-azepan-1-yl), 2-Hydroxy-6-oxo-piperazin-1-yl, 2-Methoxy- 6-oxo-piperazin-1-yl, 2-Aza-bicyclo[2.2.2]-octan-3-on-2-yl, 5,6- Dihydro-1*H*-pyrimidin-2-oxo-1-yl, 2-Oxo-[1,3]oxazinan-3-yl oder 4*H*-[1,4]Oxazin-4-yl, oder N(R²)₂ und falls Y = Piperidin-1,4-diyl, auch R² oder Cycloalkyl,
- A: unverzweigtes oder verzweigtes Alkyl mit 1-10 C-Atomen, worin eine oder zwei CH₂-Gruppen durch O- oder S-Atome und/oder durch -CH=CH-Gruppen und/oder auch 1-7 H-Atome durch F ersetzt sein können,
- Ar: unsubstituiertes oder ein-, zwei- oder dreifach durch Hal, A, OR², SO₂A, SO₂NH₂, COOR² oder CN substituiertes Phenyl,
- Hal: F, Cl, Br oder I,
- m: 1 oder 2,
- n: 0, 1 oder 2 bedeuten,
sowie ihre pharmazeutisch verwendbaren Salze, Solvate und Stereoisomere, einschließlich deren Mischungen in allen Verhältnissen.

Gegenstand der Erfindung sind auch die optisch aktiven Formen, die Racemate, die Diastereomeren sowie die Hydrate und Solvate, z.B. Alkoholate, dieser Verbindungen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie Faktor Xa inhibierende Eigenschaften und können daher zur Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie und Claudicatio intermittens eingesetzt werden.

Die erfindungsgemäßen Verbindungen der Formel I sind weiterhin Inhibitoren der Gerinnungsfaktoren Faktor Vlla, Faktor IXa und Thrombin der Blutgerinnungskaskade sein.

Andere aromatische Amide sind in der WO 02/48099 A, WO 99/00121 und in der WO 00/39118 beschrieben. Aromatische Amidinderivate mit antithrombotischer Wirkung sind z.B. aus der EP 0 540 051 B1 bekannt. Cyclische Guanidine zur Behandlung thromboembolischer Erkrankungen sind z.B. in der WO 97/08165 beschrieben. Aromatische Heterocyclen mit Faktor Xa inhibitorischer Aktivität sind z.B. aus der WO 96/10022 bekannt. Substituierte N-[(Aminoiminomethyl)phenylalkyl]-azaheterocyclylamide als Faktor Xa Inhibitoren sind in WO 96/40679 beschrieben.

Der antithrombotische und antikoagulierende Effekt der erfindungsgemäßen Verbindungen wird auf die inhibierende Wirkung gegenüber der aktivierten Gerinnungsprotease, bekannt unter dem Namen Faktor Xa, oder auf die Hemmung anderer aktivierter Serinproteasen wie Faktor Vlla, Faktor IXa oder Thrombin zurückgeführt.

Faktor Xa ist eine der Proteasen, die in den komplexen Vorgang der Blutgerinnung involviert ist. Faktor Xa katalysiert die Umwandlung von Prothrombin in Thrombin. Thrombin spaltet Fibrinogen in Fibrinmonomere, die nach Quervernetzung elementar zur Thrombusbildung beitragen. Eine Aktivierung von Thrombin kann zum Auftreten von thromboembolischen Erkrankungen führen. Eine Hemmung von Thrombin kann jedoch die in die Thrombusbildung involvierte Fibrinbildung inhibieren.
Die Messung der Inhibierung von Thrombin kann z.B. nach der Methode von G. F. Cousins et al. in Circulation 1996, 94, 1705-1712 erfolgen.

Eine Inhibierung des Faktors Xa kann somit verhindern, daß Thrombin gebildet wird.
Die erfindungsgemäßen Verbindungen der Formel I sowie ihre Salze greifen durch Inhibierung des Faktors Xa in den Blutgerinnungsprozeß ein und hemmen so die Entstehung von Thromben.

Die Inhibierung des Faktors Xa durch die erfindungsgemäßen Verbindungen und die Messung der antikoagulierenden und antithrombotischen Aktivität kann nach üblichen in vitro- oder in vivo-Methoden ermittelt werden. Ein geeignetes Verfahren wird z.B. von J. Hauptmann et al. in Thrombosis and Haemostasis 1990, 63, 220-223 beschrieben.

Die Messung der Inhibierung von Faktor Xa kann z.B. nach der Methode von T. Hara et al. in Thromb. Haemostas. 1994, 71, 314-319 erfolgen.

Der Gerinnungsfaktor Vlla initiiert nach Bindung an Tissue Faktor den extrinsischen Teil der Gerinnungskaskade und trägt zur Aktivierung des Faktors X zu Faktor Xa bei. Eine Inhibierung von Faktor Vlla verhindert somit die Entstehung des Faktors Xa und damit eine nachfolgende Thrombinbildung.
Die Inhibierung des Faktors Vlla durch die erfindungsgemäßen Verbindungen und die Messung der antikoagulierenden und antithrombotischen Aktivität kann nach üblichen in vitro- oder in vivo-Methoden ermittelt werden. Ein übliches Verfahren zur Messung der Inhibierung von Faktor VIIa wird z.B. von H. F. Ronning et al. in Thrombosis Research 1996, 84, 73-81 beschrieben.

Der Gerinnungsfaktor IXa wird in der intrinsischen Gerinnungskaskade generiert und ist ebenfalls an der Aktivierung von Faktor X zu Faktor Xa beteiligt. Eine Inhibierung von Faktor IXa kann daher auf andere Weise verhindern, daß Faktor Xa gebildet wird.
Die Inhibierung von Faktor IXa durch die erfindungsgemäßen Verbindungen und die Messung der antikoagulierenden und antithrombotischen Aktivität kann nach üblichen in vitro- oder in vivo-Methoden ermittelt werden. Ein geeignetes Verfahren wird z.B. von J. Chang et al. in Journal of Biological Chemistry 1998, 273, 12089-12094 beschrieben.

Die erfindungsgemäßen Verbindungen können weiterhin zur Behandlung von Tumoren, Tumorerkrankungen und/oder Tumormetastasen verwendet werden.

Ein Zusammenhang zwischen dem Tissuefaktor TF / Faktor Vlla und der Entwicklung verschiedener Krebsarten wurde von T.Taniguchi und N.R.Lemoine in Biomed. Health Res. (2000), 41 (Molecular Pathogenesis of Pancreatic Cancer), 57-59, aufgezeigt.

Die im nachfolgenden aufgeführten Publikationen beschreiben eine antitumorale Wirkung von TF-VII und Faktor Xa Inhibitoren bei verschiedenen Tumorarten:
K.M. Donnelly et al. in Thromb. Haemost. 1998; 79: 1041-1047;
E.G. Fischer et al. in J. Clin. Invest. 104: 1213-1221 (1999);
B.M. Mueller et al. in J. Clin. Invest. 101: 1372-1378 (1998);
M.E. Bromberg et al. in Thromb. Haemost. 1999; 82: 88-92

Die Verbindungen der Formel I können als Arzneimittelwirkstoffe in der Human- und Veterinärmedizin eingesetzt werden, insbesondere zur Behandlung und Verhütung von thromboembolischen Erkrankungen wie Thrombose, myocardialem Infarkt, Arteriosklerose, Entzündungen, Apoplexie, Angina pectoris, Restenose nach Angioplastie, Claudicatio intermittens, venöse Thrombose, pulmonale Embolie, arterielle Thrombose, myocardiale Ischämie, instabile Angina und auf Thrombose basierender Schlaganfall.
Die erfindungsgemäßen Verbindungen werden auch zur Behandlung oder Prophylaxe von atherosklerotischen Erkrankungen wie koronarer arterieller Erkrankung, cerebraler arterieller Erkrankung oder peripherer arterieller Erkrankung eingesetzt.
Die Verbindungen werden auch in Kombination mit anderen Thrombolytika bei myocardialem Infarkt eingesetzt, ferner zur Prophylaxe zur Reocclusion nach Thrombolyse, percutaner transluminaler Angioplastie (PTCA) und koronaren Bypass-Operationen.
Die erfindungsgemäßen Verbindungen werden ferner verwendet zur Prävention von Rethrombose in der Mikrochirurgie, ferner als Antikoagulantien im Zusammenhang mit künstlichen Organen oder in der Hämodialyse. Die Verbindungen finden ferner Verwendung bei der Reinigung von Kathetern und medizinischen Hilfsmitteln bei Patienten *in vivo,* oder als Antikoagulantien zur Konservierung von Blut, Plasma und anderen Blutprodukten *in vitro.* Die erfindungsgemäßen Verbindungen finden weiterhin Verwendung bei solchen Erkrankungen, bei denen die Blutkoagulation entscheidend zum Erkrankungsverlauf beiträgt oder eine Quelle der sekundären Pathologie darstellt, wie z.B. bei Krebs einschließlich Metastasis, entzündlichen Erkrankungen einschließlich Arthritis, sowie Diabetes.

Die erfindungsgemäßen Verbindungen finden weiterhin Verwendung zur Behandlung von Migräne (F.Morales-Asin et al., Headache, 40, 2000, 45-47).

Bei der Behandlung der beschriebenen Erkrankungen werden die erfindungsgemäßen Verbindungen auch in Kombination mit anderen thrombolytisch wirksamen Verbindungen eingesetzt, wie z.B. mit dem "tissue plasminogen activator" t-PA, modifiziertem t-PA, Streptokinase oder Urokinase. Die erfindungsgemäßen Verbindungen werden mit den anderen genannten Substanzen entweder gleichzeitig oder vorher oder nachher gegeben.
Besonders bevorzugt ist die gleichzeitige Gabe mit Aspirin, um ein Neuauftreten der Thrombenbildung zu verhindern.
Die erfindungsgemäßen Verbindungen werden auch verwendet in Kombination mit Blutplättchen-Glycoprotein-Rezeptor (IIb/IIIa)-Antagonisten, die die Blutplättchenaggregation inhibieren.

Gegenstand der Erfindung sind die Verbindungen der Formel I und ihre Salze sowie ein Verfahren zur Herstellung von Verbindungen der Formel I nach Anspruch 1 sowie ihrer Salze, dadurch gekennzeichnet, daß man
a) eine Verbindung der Formel II 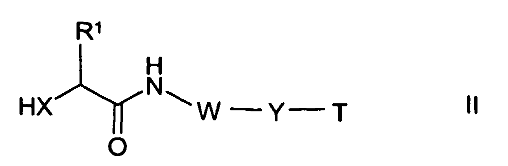 worin
   R¹, T, W, X und Y die in Anspruch 1 angegebene Bedeutung haben,
   mit einer Verbindung der Formel III

   D-N=C=O III

   worin
   D die in Anspruch 1 angegebene Bedeutung hat,
   umsetzt,
   oder
b) eine Verbindung der Formel IV

   H₂N—W—Y—T IV,

   worin W, Y und T die in Anspruch 1 angegebene Bedeutung haben,
   mit einer Verbindung der Formel V 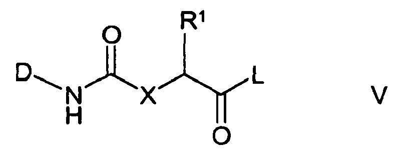 worin
   - L: Cl, Br, I oder eine freie oder reaktionsfähig funktionell abgewandelte OH-Gruppe bedeutet und
   - R¹, X und D: die in Anspruch 1 angegebenen Bedeutungen haben,
   umsetzt,
   oder
c) einen Rest R¹ in einen anderen Rest R¹ umwandelt, indem man den Rest R¹ oxidiert
und/oder eine Base oder Säure der Formel I in eines ihrer Salze umwandelt.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.

Gegenstand der Erfindung sind auch Mischungen der erfindungsgemäßen Verbindungen der Formel I, z.B. Gemische zweier Diastereomerer z.B. im Verhältnis 1:1, 1:2, 1:3, 1:4, 1:5, 1:10, 1:100 oder 1 :1000.
Besonders bevorzugt handelt es sich dabei um Mischungen stereoisomerer Verbindungen.

Für alle Reste, die mehrfach auftreten, gilt, daß deren Bedeutungen unabhängig voneinander sind.

Vor- und nachstehend haben die Reste bzw. Parameter R¹, D, W, T, X und Y die bei der Formel I angegebenen Bedeutungen, falls nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2-, 2,3- oder 3,3-Dimethylbutyl, 1- oder 2-Ethylbutyl, 1-Ethyl-1-methylpropyl, 1-Ethyl-2-methylpropyl, 1,1,2-oder 1,2,2-Trimethylpropyl, weiter bevorzugt z.B. Trifluormethyl.
A bedeutet ganz besonders bevorzugt Alkyl mit 1-6 C-Atomen, vorzugsweise Methyl, Ethyl, Propyl, Isopropyl, Butyl, Isobutyl, sek.-Butyl, tert.-Butyl, Pentyl, Hexyl oder Trifluormethyl.

Cycloalkyl hat 3-7 C-Atome und bedeutet vorzugsweise Cyclopropyl, Cyclobutyl, Cylopentyl, Cyclohexyl oder Cycloheptyl.
Hal bedeutet vorzugsweise F, CI oder Br, aber auch I.
Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Methoxyphenyl, o-, m- oder p-Ethoxyphenyl, o-, m- oder p-Ethoxycarbonylphenyl, o-, m- oder p-Fluorphenyl, o-, m- oder p-Bromphenyl, o-, m- oder p- Chlorphenyl, o-, m- oder p-(Methylsulfonyl)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Fluor-4-methoxyphenyl oder 2,5-Dimethyl-4-chlorphenyl.

Ar bedeutet vorzugsweise z.B. unsubstituiertes oder ein-, zwei- oder dreifach durch Hal, A, OR², SO₂A, COOR² oder CN substituiertes Phenyl. Ar bedeutet insbesondere bevorzugt z.B. unsubstituiertes oder ein- oder zweifach durch Hal, A, OA, SO₂A, SO₂NH₂, COOR² oder CN substituiertes Phenyl, wie z.B. Phenyl, 2-Methylsulfonylphenyl, 2-Aminosulfonylphenyl, 2-, 3- oder 4-Chlorphenyl, 4-Methylphenyl, 4-Bromphenyl, 3-Fluor-4-methoxyphenyl, 4-Trifluormethoxyphenyl, 4-Ethoxyphenyl, 2-Methoxyphenyl, 3-Cyanphenyl oder 4-Ethoxycarbonylphenyl.
Ganz besonders bevorzugt bedeutet Ar unsubstituiertes Phenyl, 4-Chlorphenyl oder 2-Methylsulfonylphenyl.

Y bedeutet vorzugsweise Ar-diyl, besonders bevorzugt unsubstituiertes oder einfach durch A, CI oder F substituiertes 1,4-Phenylen.
Y bedeutet insbesondere unsubstituiertes oder einfach durch Methyl, Ethyl, Propyl, CI oder F substituiertes 1,3- oder 1,4-Phenylen.
Y bedeutet ganz besonders bevorzugt unsubstituiertes oder einfach durch A subsituiertes Phenylen.

W fehlt vorzugsweise.

D bedeutet insbesondere einfach durch Hal substituiertes Phenyl.

D bedeutet ganz besonders bevorzugt 4-Chlorphenyl.

R¹ bedeutet vorzugsweise z.B. Alkyl mit 1, 2, 3, 4, 5 oder 6 C-Atomen, das einfach durch S(O)ₘR², SO₂N(R²)_{2,} SO₃R², S(=O)(=NR²)R², NR²SO₂R², OSO₂R², OSO₂N(R²)₂ oder PO(OR²)₂ substituiert ist, wobei R² vorzugsweise z.B. H oder Alkyl mit 1, 2, 3, 4, 5 oder 6 C-Atomen bedeutet.

R² bedeutet vorzugsweise z.B. H oder Alkyl mit 1, 2, 3, 4, 5 oder 6 C-Atomen, ganz besonders bevorzugt H.
n bedeutet vorzugsweise 0 oder 1.
m bedeutet vorzugsweise 2.

T bedeutet vorzugsweise einen einkernigen gesättigten oder ungesättigten Heterocyclus mit 1 bis 2 N- und/oder O- Atomen, der ein- oder zweifach durch Carbonylsauerstoff, OH oder OA substituiert sein kann.
T bedeutet besonders bevorzugt z.B. Piperidin-1-yl, 2-Oxo-piperidin-1-yl, 2-Oxo-pyrrolidin-1-yl, Pyrrolidin-1-yl, 3-Oxo-morpholin-4-yl, Morpholin-4-yl, 4-Oxo-1*H*-pyridin-1-yl, 2,6-Dioxo-piperidin1-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2*H*-pyridazin-2-yl, 2-Caprolactam-1-yl (= 2-Oxo-azepan-1-yl), 2-Hydroxy-6-oxo-piperazin-1-yl, 2-Methoxy-6-oxo-piperazin-1-yl, 2-Aza-bicyclo[2.2.2]-octan-3-on-2-yl, 5,6-Dihydro-1*H*-pyrimidin-2-oxo-1-yl, 2-Oxo-[1,3]oxazinan-3-yl oder 4*H*-[1,4]Oxazin-4-yl.

Ganz besonders bevorzugt ist 2-Oxo-piperidin-1-yl, 2-Oxo-pyrrolidin-1-yl, 3-Oxo-morpholin-4-yl, Morpholin-4-yl, Piperidin-1-yl, Pyrrolidin-1-yl oder 3-Oxo-2*H*-pyridazin-2-yl.

Wenn Y 1,4-Piperidinyl bedeutet, dann ist T vorzugsweise auch z.B. R², vorzugsweise z.B. H oder Alkyl mit 1, 2, 3, 4, 5 oder 6 C-Atomen oder Cycloalkyl wie z.B. Isopropyl, Cyclopentyl oder Cyclohexyl.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Eine bevorzugte Gruppe von Verbindungen kann durch die folgende Teilformel Ia ausgedrückt werden, die der Formel I entspricht und worin die nicht näher bezeichneten Reste die bei der Formel I angegebene Bedeutung haben, worin jedoch
in la
- D: einfach durch Hal substituiertes Phenyl,
- R¹: Alkyl mit 1, 2, 3, 4, 5 oder 6 C-Atomen, das einfach durch S(O)ₘR², SO²N(R²)₂, SO₃R², S(=O)(=NR²)R², NR²O₂R², OSO₂R², OSO₂N(R²)₂ oder PO(OR²)₂ substituiert ist,
- R²: H oder Alkyl mit 1, 2, 3, 4, 5 oder 6 C-Atomen,
- W: -(CH₂)ₙ-,
- X: NH oder O,
- Y: unsubstituiertes oder einfach durch A substituiertes Phenylen,
- T: Piperidin-1-yl, 2-Oxo-piperidin-1-yl, 2-Oxo-pyrrolidin-1-yl, Pyrrolidin-1-yl, 3-Oxo-morpholin-4-yl, Morpholin-4-yl, 4- Oxo-1*H*-pyridin-1-yl, 2,6-Dioxo-piperidin1-yl, 2-Oxo- piperazin-1-yl, 2,6-Dioxo-piperazin-1-yl, 2,5-Dioxo- pyrrolidin-1-yl, 2-Oxo-1,3-oxazolidin-3-yl, 3-Oxo-2*H*- pyridazin-2-yl, 2-Caprolactam-1-yl (= 2-Oxo-azepan-1-yl), 2-Hydroxy-6-oxo-piperazin-1-yl, 2-Methoxy-6-oxo- piperazin-1-yl, 2-Aza-bicyclo[2.2.2]-octan-3-on-2-yl, 5,6- Dihydro-1*H*-pyrimidin-2-oxo-1-yl, 2-Oxo-[1,3]oxazinan-3-yl oder 4*H*-[1,4]Oxazin-4-yl, oder N(R²)₂ und falls Y = Piperidin-1,4-diyl, auch R² oder Cycloalkyl,
- A: unverzweigtes oder verzweigtes Alkyl mit 1-10 C-Atomen, worin eine oder zwei CH₂-Gruppen durch O- oder S-Atome und/oder durch -CH=CH-Gruppen und/oder auch 1-7 H- Atome durch F ersetzt sein können,
- Hal: F, CI, Br oder I,
- m: 1 oder 2,
- n: 0, 1 oder 2
bedeuten;
sowie ihre pharmazeutisch verwendbaren Salze, Solvate und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Verbindungen der Formel I können vorzugsweise erhalten werden, indem man Verbindungen der Formel II mit Verbindungen der Formel III umsetzt. Die Umsetzung erfolgt in der Regel in einem inerten Lösungsmittel, in Gegenwart eines säurebindenden Mittels vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums. Auch der Zusatz einer organischen Base wie Triethylamin, Dimethylanilin, Pyridin oder Chinolin kann günstig sein. Die Reaktionszeit liegt je nach den angewendeten Bedingungen zwischen einigen Minuten und 14 Tagen, die Reaktionstemperatur zwischen etwa 0° und 150°, normalerweise zwischen 20° und 130°.

Als inerte Lösungsmittel eignen sich z.B. Wasser; Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Die Ausgangsverbindungen der Formeln II und III sind in der Regel bekannt. Sind sie neu, so können sie aber nach an sich bekannten Methoden hergestellt werden.

Verbindungen der Formel I können auch erhalten werden, indem man Verbindungen der Formel IV mit Verbindungen der Formel V umsetzt.

In den Verbindungen der Formel V bedeutet L vorzugsweise CI, Br, I oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).
Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.
Aktivierte Ester werden zweckmäßig in situ gebildet, z. B. durch Zusatz von HOBt oder N-Hydroxysuccinimid.

Die Umsetzung erfolgt in der Regel in einem inerten Lösungsmittel, in Gegenwart eines säurebindenden Mittels vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums. Auch der Zusatz einer organischen Base wie Triethylamin, Dimethylanilin, Pyridin oder Chinolin oder eines Überschusses der Aminkomponente der Formel IV kann günstig sein. Die Reaktionszeit liegt je nach den angewendeten Bedingungen zwischen einigen Minuten und 14 Tagen, die Reaktionstemperatur zwischen etwa 0° und 150°, normalerweise zwischen 20° und 130°.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol;Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Verbindungen der Formel I können auch erhalten werden, indem man Verbindungen der Formel I aus einem ihrer funktionellen Derivate durch Behandeln mit einem solvolysierenden oder hydrogenolysierenden Mittel in Freiheit setzt.

Bevorzugte Ausgangsstoffe für die Solvolyse bzw. Hydrogenolyse sind solche, die sonst der Formel I *e*ntsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I entsprechen, jedoch anstelle einer Gruppe -COOH eine Gruppe -COOR" tragen, worin R" eine Hydroxyschutzgruppe bedeutet.
Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC (tert.-Butyloxycarbonyl), 2-lodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ, Benzyl oder die Freisetzung der Amidinogruppe aus ihrem Oxadiazolderivat)) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammoniumformiat (anstelle von Wasserstoff) an Pd/C in Methanol/DMF bei 20-30°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Trifluormethylbenzol, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid, N-Methylpyrrolidon (NMP) oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Die Biphenyl-SO₂NH₂-Gruppe wird vorzugsweise in Form ihres tert.-Butylderivates eingesetzt. Die Abspaltung der tert.-Butylgruppe erfolgt z.B. mit TFA mit oder ohne Zusatz eines inerten Lösungsmittels, vorzugsweise unter Zusatz einer geringen Menge an Anisol (1-10 Vol %).

Es ist ferner möglich, eine Verbindung der Formel I in eine andere Verbindung der Formel I umzuwandeln, indem man einen oder mehrere Rest(e) R¹, D, E und/oder W in einen oder mehrere Rest(e) R¹, D, Y, und/oder T umwandelt, z.B. indem man eine Aminogruppe acyliert oder Nitrogruppen (beispielsweise durch Hydrierung an Raney-Nickel oder Pd-Kohle in einem inerten Lösungsmittel wie Methanol oder Ethanol) zu Aminogruppen reduziert.

Ester können z.B. mit Essigsäure oder mit NaOH oder KOH in Wasser, Wasser-THF oder Wasser-Dioxan bei Temperaturen zwischen 0 und 100° verseift werden.

Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und /oder in Gegenwart einer Base wie Triethylamin oder Pyridin bei Temperaturen zwischen -60 und +30°.

Bedeutet Y 1,4-Piperidinyl, so kann die Alkylierung des Piperidin-Stickstoffs nach üblichen Methoden der reduktiven Aminierung erfolgen.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können Verbindungen der Formel I mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden.
Auch physiologisch unbedenkliche organische Basen, wie z.B. Ethanolamin können verwendet werden.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktive Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine Verbindung der Formel I und/oder ihre pharmazeutisch verwendbaren Solvate und Stereoisomere, einschließlich deren Mischungen in allen Verhältnissen, sowie gegebenenfalls Träger- und/oder Hilfsstoffe.

Diese Arzneimittel können in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder oder auch als Nasenspray. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie, Claudicatio intermittens, Tumoren, Tumorerkrankungen und/oder Tumormetastasen verwendet werden.

Dabei werden die erfindungsgemäßen Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Gegenstand der Erfindung sind ferner Arzneimittel enthaltend mindestens eine Verbindung der Formel I und/oder ihre pharmazeutisch verwendbaren Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Solvate und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Solvate und Stereoisomere, einschließlich deren Mischungen in allen Verhältnissen,
und einer wirksamen Menge eines weiteren Arzneimittelwirkstoffs gelöst oder in lyophylisierter Form vorliegt.

Gegenstand der Erfindung ist ferner die Verwendung von Verbindungen der Formel I und/oder ihrer pharmazeutisch verwendbaren Solvate und Stereoisomere, einschließlich deren Mischungen in allen Verhältnissen, zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen, in Kombination mit mindestens einem weiteren Arzneimittelwirkstoff.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
- Massenspektrometrie (MS):: EI (Elektronenstoß-lonisation) M⁺
ESI (Electrospray lonization) (M+H)⁺
FAB (Fast Atom Bombardment) (M+H)⁺

### Beispiel 1 (Vergleichsbeispiel)

Die Herstellung von 2-[3-(4-Ghlorphenyl)-ureido]-*N*-[4-(2-oxo-2*H*-pyridin-1-yl)-phenyl]-4-methansulfonyl-butyramid erfolgt analog nachstehendem Schema:

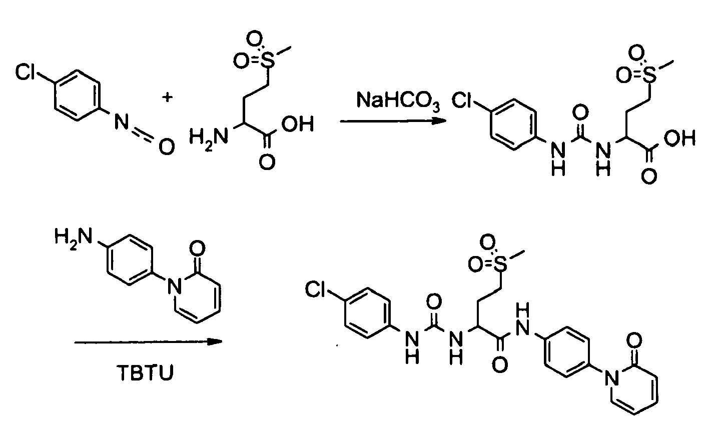

1.1 Eine Lösung von 9.24 g (110 mmol) Natriumhydrogencarbonat und 5.0 g (27.6 mmol) 2-Amino-4-methansulfonylbuttersäure in 50 ml Wasser wird auf 80° C erhitzt und 8.45 g (55.0 mmol) 4-Chlorphenylisocyanat zugegeben. Das Reaktionsgemisch wird 1 Stunde bei dieser Temperatur gerührt. Man lässt abkühlen und filtriert den entstandenen Niederschlag ab. Das Filtrat wird mit 1 N HCI angesäuert und der entstandene Niederschlag abfiltriert und getrocknet: 2-[3-(4-Chlorphenyl)-ureido]-4-methansulfonylbuttersäure als farbloser Feststoff; ESI 335.

1.2 Eine Lösung von 167 mg (0.500 mmol) 2-[3-(4-Chlorphenyl)-ureido]-4-methansulfonylbuttersäure und 93.1 mg (0.500 mmol) 1-(4-Amino-phenyl)-1*H*-pyridin-2-on in 1 ml DMF wird mit 209 mg (0.650 mmol) [(Benzotriazol-1-yloxy)-dimethylamino-methylen]-dimethylammoniumtetrafluoroborat (TBTU) versetzt und 24 Stunden bei Raumtemperatur gerührt. Das Reaktionsgemisch wird auf gesättigte Natriumhydrogencarbonatlösung gegeben, der entstandenene Niederschlag abfiltriert und getrocknet: 2-[3-(4-Chlorphenyl)-ureido]-*N*-[4-(2-oxo-2*H*-pyridin-1-yl)-phenyl]-4-methansulfonyl-butyramid ("1A") als farbloser Feststoff; ESI 503.

Analog werden die nachstehenden Verbindungen erhalten
2-[3-(4-Chlorphenyl)-ureido]-*N*-[4-(2-oxo-2*H*-pyrazin-1-yl)-phenyl]-4-methansulfonyl-butyramid ("1 B"), ESI 504;
2-[3-(4-Chlorphenyl)-ureido]-*N*-[4-(3-oxo-morpholin-4-yl)-phenyl]-4-methansulfonyl-butyramid ("1C"), ESI 509;
(R)-2-[3-(4-Chlorphenyl)-ureido]-*N*-[4-(3-oxo-morpholin-4-yl)-phenyl]-4-methansulfonyl-butyramid ("2C"), ESI 509;
(R)-2-[3-(4-Chlorphenyl)-ureido]-*N*-(4-(2-oxo-2*H*-pyridin-1-yl)-phenyl]-4-methansulfonyl-butyramid ("2D"), ESI 503;
(R)-2-[3-(4-Chlorphenyl)-ureido]-*N*-[3-methyl-4-(3-oxo-morpholin-4-yl)-phenyl]-4-methansulfonyl-butyramid, ESI 523;

### Beispiel 2

Die Herstellung von (R)-2-[3-(4-Chlorphenyl)-ureido]-*N*-[4-(2-oxo-2*H-*pyridin-1-yl)-phenyl]-3-methansulfonyl-propionamid erfolgt analog nachstehendem Schema:

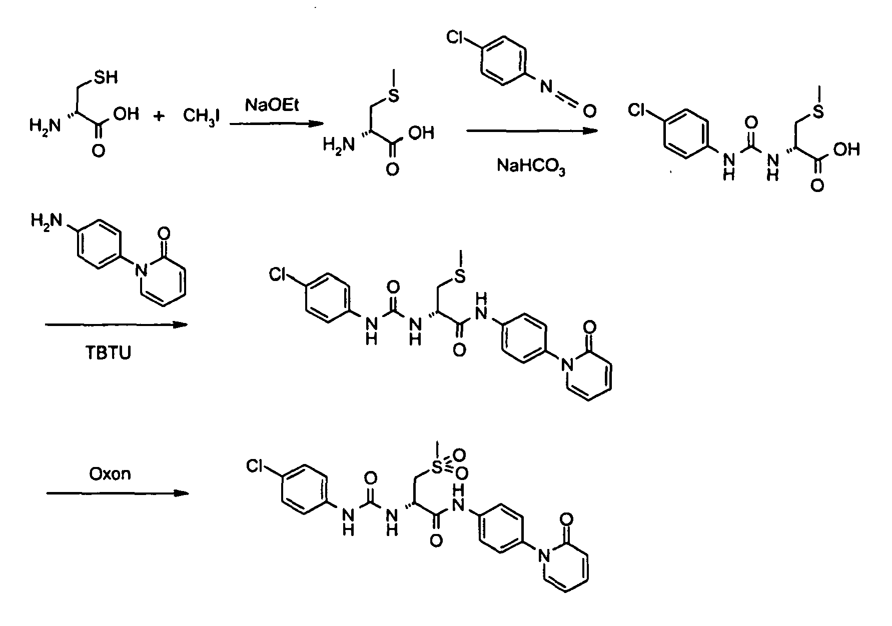

2.1 Eine Suspension von 25 g (142 mmol) D-Cystein-hydrochlorid Hydrat in 350 ml Ethanol wird nach und nach unter Rühren mit 13.0 g (565 mmol) Natrium versetzt. Nachdem das Natrium aufgelöst ist, wird 10.0 ml (160 mmol) Methyliodid zugetropft. Nach weiteren 30 min Rühren bei Raumtemperatur wird zu dem Reaktionsgemisch Wasser gegeben, bis eine klare Lösung entsteht. Dann wird Essigsäure bis zum Erreichen eines pH von 6 zugegeben. Das Reaktionsgemisch wird im Vakuum auf ein Volumen von ca. 200 ml eingedampft und auf 5° C abgekühlt. Der entstandene Niederschlag wird abfiltriert: (R)-2-Amino-3-methylsulfanylpropionsäure im Gemisch mit Natriumacetat (Gewichtsverhältnis 35 : 65) als farbloser Feststoff; ESI 136.

2.2 Eine Lösung von 18.0 g (214 mmol) Natriumhydrogencarbonat und 13.8 g (35.7 mmol) 35%ige (R)-2-Amino-3-methylsulfanylpropionsäure in 200 ml Wasser wird auf 80° C erhitzt und 11.0 g (71.6 mmol) 4-Chlorphenylisocyanat zugegeben. Das Reaktionsgemisch wird 1 Stunde bei dieser Temperatur gerührt. Man lässt abkühlen und filtriert den entstandenen Niederschlag ab. Das Filtrat wird mit 1 N HCI angesäuert und der entstandene Niederschlag abfiltriert und getrocknet: (R)-2-[3-(4-Chlorphenyl)-ureido]-3-methylsulfanyl-propionsäure als leicht grünlicher Feststoff; ESI 289.

2.3 Eine Lösung von 1.00 g (3.46 mmol) : (R)-2-[3-(4-Chlorphenyl)-ureido]-3-methylsulfanyl-propionsäure und 640 mg (3.44 mmol) 1-(4-Amino-phenyl)-1*H*-pyridin-2-on in 5 ml DMF wird mit 1.440 g (4.49 mmol) [(Benzotriazol-1-yloxy)-dimethylamino-methylen]-dimethylammoniumtetrafluoroborat (TBTU) versetzt und 24 Stunden bei Raumtemperatur gerührt. Das Reaktionsgemisch wird auf gesättigte Natriumhydrogencarbonatlösung gegeben, der entstandenene Niederschlag abfiltriert und getrocknet: (R)-2-[3-(4-Chlorphenyl)-ureido]-3-methylsulfanyl-*N*-[4-(2-oxo-2*H-*pyridin-1-yl)-phenyl]-propionamid als farbloser Feststoff; ESI 457.

2.4 Eine Lösung von 200 mg (0.438 mmol) (R)-2-[3-(4-Chlorphenyl)-ureido]-3-methylsulfanyl-*N-*[4-(2-oxo-2*H-*pyridin-1-yl)-phenyl]-propionamid in 10 ml Methanol wird mit einer Lösung von 400 mg Oxon in 6 ml Wasser versetzt und das Reaktionsgemisch 48 Stunden bei Raumtemperatur gerührt. Das Reaktionsgemisch wird auf Wasser gegeben und der entstandene Niederschlag abfiltriert und getrocknet: (R)-2-[3-(4-Chlorphenyl)-ureido]-*N*-[4-(2-oxo-2*H*-pyridin-1-yl)-phenyl]-3-methansulfonyl-propionamid als farbloser Feststoff ("2A"); ESI 489.

Analog werden die nachstehenden Verbindungen erhalten
(S)-2-[3-(4-Chlorphenyl)-ureido]-*N*-[4-(2-oxo-2*H*-pyridin-1-yl)-phenyl]-3-methansulfonyl-propionamid,
(S)-2-[3-(4-Chlorphenyl)-ureido]-*N*-[4-(3-oxo-morpholin-4-yl)-phenyl]-3-methansulfonyl-propionamid, ("2B"), ESI 495;
(R)-2-[3-(4-Chlorphenyl)-ureido]-*N*-[3-methyl-4-(3-oxo-morpholin-4-yl)-phenyl]-3-methansulfonyl-propionamid, ESI 509;
(R)-2-[3-(4-Chlorphenyl)-ureido]-*N*-[4-(2-oxo-[1,3]oxazinan-3-yl)-phenyl]-3-methansulfonyl-propionamid, ESI 495.

### Beispiel 3

Die Herstellung von (R)-2-[*N*-(4-Chlorphenyl)-carbamoyloxy]-*N*-[4-(3-oxo-morpholin-4-yl)-phenyl]-3-methansulfonyl-propionamid erfolgt analog nachstehendem Schema:

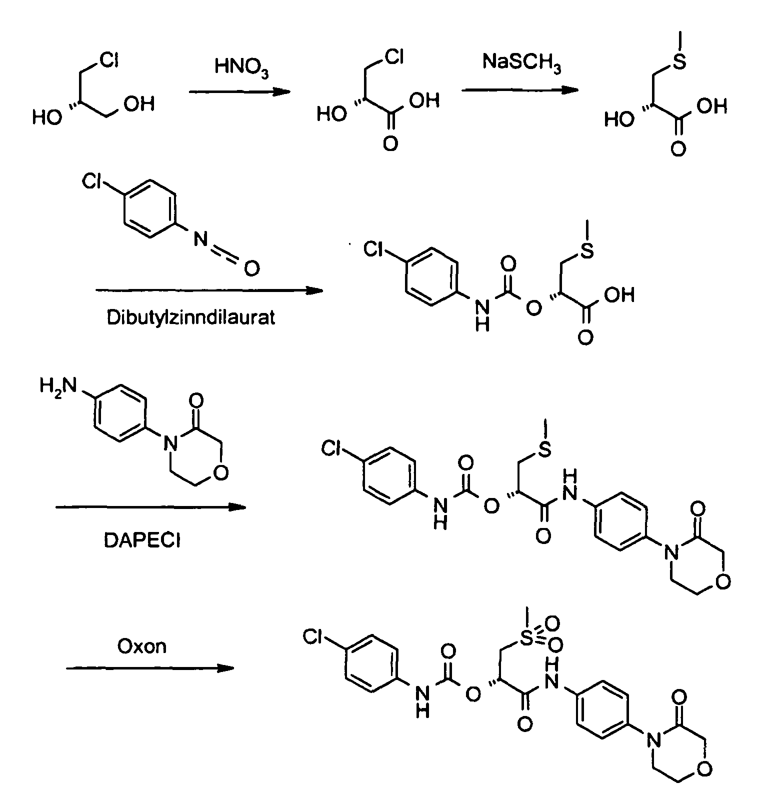

3.1 24 g (217 mmol) (S)-3-Chlor-1,2-propandiol wird in 60 ml auf 0° C gehaltener 65%iger Salpetersäure gelöst. Die Lösung wird 30 min auf 70° C und anschließend 15 min auf 100° C erhitzt. Man lässt das Reaktionsgemisch abkühlen, gibt 15 g Natriumhydrogencarbonat zu und extrahiert mit tert.-Butylmethylether. Die organische Phase wird über Natriumsulfat getrocknet, eingedampft und der Rückstand aus Chloroform umkristallisiert: (R)-3-Chlor-2-hydroxypropionsäure als farblose Nadeln vom Schmpkt. 93° C, ESI 125.

3.2 Eine Lösung von 5.00 g (40.2 mmol) (R)-3-Chlor-2-hydroxypropionsäure in 80 ml Methanol wird mit 11.2 g (160 mmol) Natriummethanthiolat versetzt und 18 Stunden zum Sieden erhitzt. Das Reaktionsgemisch wird filtriert und das Filtrat eingedampft. Der Rückstand wird mit 2 N HCI angesäuert und mit Ethylacetat extrahiert. Die organische Phase wird eingedampft: (R)-2-Hydroxy-3-methylsulfanylpropionsäure als gelbliches Öl; ESI 137.

3.3 Eine Lösung von 3.70 g (27.2 mmol) (R)-2-Hydroxy-3-methyl-sulfanylpropionsäure und 4.18 g (27.2 mmol) 4-Chlorphenylisocyanat in 50 ml Dichlormethan wird mit 400 mg (0.63 mmol) Dibutylzinndilaurat versetzt und 24 Stunden bei Raumtemperatur gerührt. Das Reaktionsgemisch wird auf Wasser gegeben und mit Ethylacetat extrahiert. Die organische Phase wird eingedampft: (R)-2-(4-Chlorphenylcarbamoyloxy)-3-methylsulfanyl-propionsäure als farbloser Feststoff; ESI 290.

3.4 Eine Lösung von 1.00 g (3.45 mmol) (R)-2-(4-Chlorphenylcarbamoyloxy)-3-methylsulfanylpropionsäure, 663 mg (3.45 mmol) 4-(4-Aminophenyl)-morpholin-3-on und 863 mg (4.50 mmol) *N*-(3-Dimethylaminopropyl)-*N*'-ethylcarbodiimidhydrochlorid (DAPECI) in 3 ml DMF wird 24 Stunden bei Raumtemperatur gerührt. Das Reaktionsgemisch wird auf gesättigte Natriumhydrogencarbonatlösung gegeben und der entstandene Niederschlag abfiltriert: (R)-2-[*N*-(4-Chlorphenyl)-carbamoyloxy]-*N*-[4-(3-oxo-morpholin-4-yl)-phenyl]-3-methansulfanyl-propionamid als farbloser Feststoff; ESI 464.

3.5 Eine Lösung von 775 mg (1.67 mmol) (R)-(4-Chlorphenyl)-carbaminsäure-2-methylsulfanyl-1-[4-(3-oxo-morpholin-4-yl)-phenylcarbamoyl]-ethylester in 50 ml Methanol wird mit einer Lösung von 2.7 g Oxon in 30 ml Wasser versetzt und das Reaktionsgemisch 24 Stunden bei Raumtemperatur gerührt. Das Reaktionsgemisch wird auf Wasser gegeben und der entstandene Niederschlag abfiltriert und getrocknet: (R)-2-[*N*-(4-Chlorphenyl)-carbamoyloxy]-*N*-[4-(3-oxo-morpholin-4-yl)-phenyl]-3-methansulfonyl-propionamid ("1 C") als farbloser Feststoff; ESI 496.

Analog erhält man
(S)-2-[*N*(4-Chlorphenyl)-carbamoyloxy]-*N*-[4-(3-oxo-morpholin-4-yl)-phenyl]-3-methansulfonyl-propionamid,
2-[*N*-(4-Chlorphenyl)-carbamoyloxy]-*N*-[4-(2-oxo-2*H*-pyridin-1-yl)-phenyl]-3-methansulfonyl-propionamid, ESI 490.

### Beispiel 4

Die Herstellung von 2-[3-(4-Chlorphenyl)-ureido]-*N*-[4-(3-oxo-morpholin-4-yl)-phenyl]-3-sulfo-propionamid erfolgt analog nachstehendem Schema:

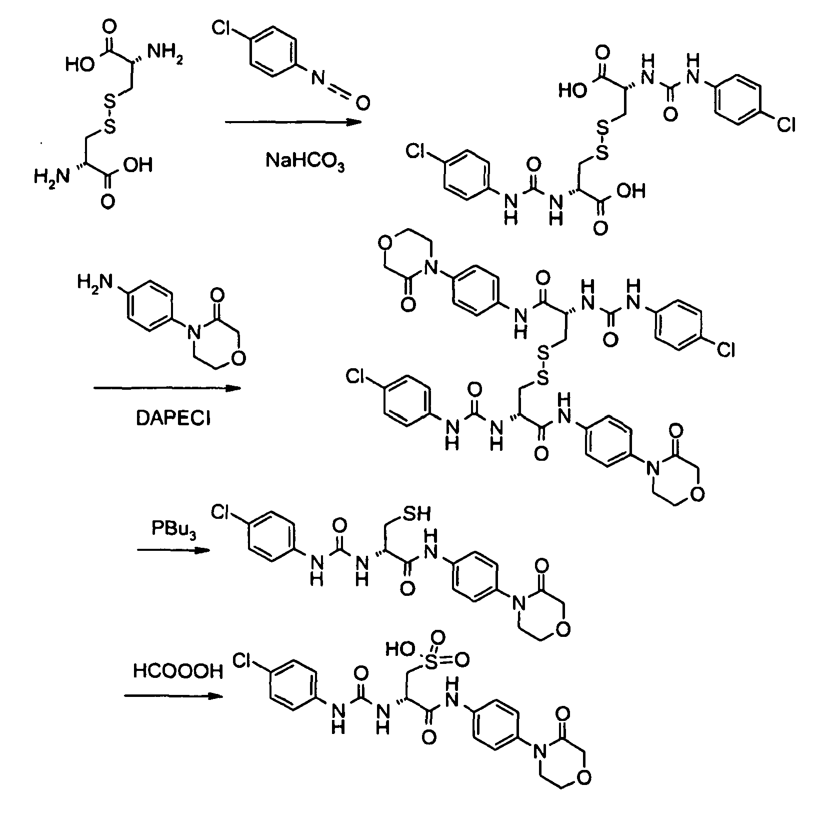

Analog erhält man 2-[3-(4-Chlorphenyl)-ureido]-*N*-[4-(2-oxo-2*H*-pyridin-1-yl)-phenyl]-3-sulfo-propionamid.

### Beispiel 5

Die Herstellung von 2-[3-(4-Chlorphenyl)-ureido]-*N*-[4-(2-oxo-piperidin-1-yl)-phenyl]-3-(dimethoxy-phosphoryl)-propionamid erfolgt analog nachstehendem Schema:

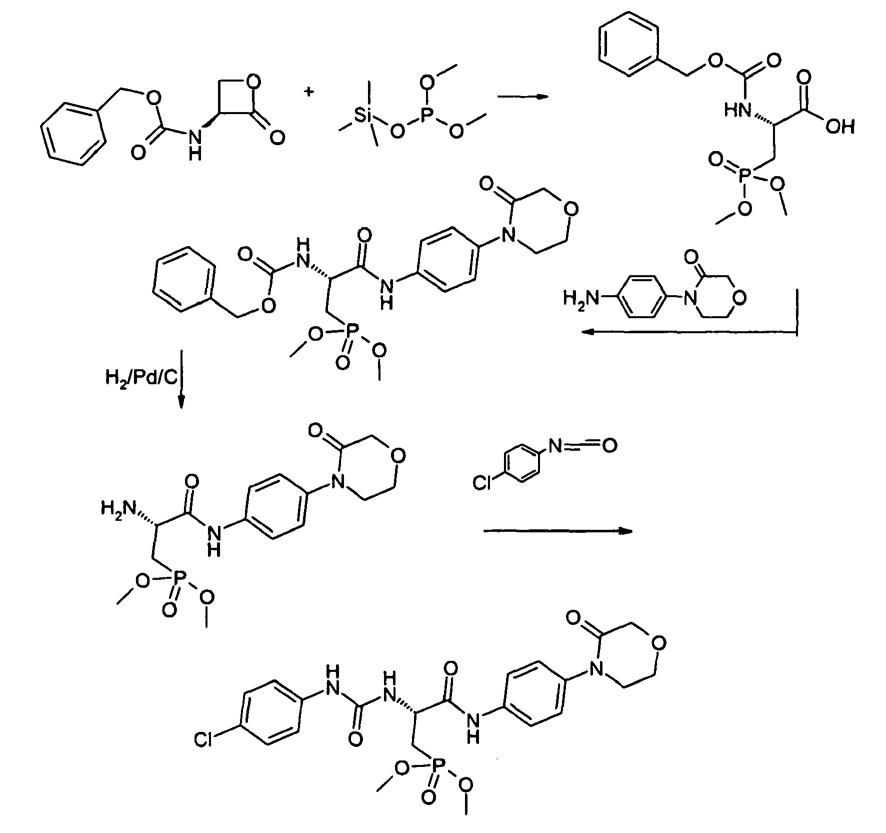

5.1 Analog zu Literatur (Lohse, P. A., Felber, R.,Tetrahedron Lett., 39; (1998); 2067-2070) erhält man aus 0.5 g (2.26 mmol) N-Benzyloxycarbonyl-L-serin β-lacton und 5 ml Dimethyltrimethylsilylphosphit 0.5 g (64.2%) (S)-2-Benzyloxycarbonylamino-3-(dimethoxy-phosphoryl)-propionsäure als farbloses Öl, ESI 331.

5.2 Analog zum Beispiel 3, 3.4 erhält man aus 0.48 g (1.45 mmol) (S)-2-Benzyloxycarbonylamino-3-(dimethoxy-phosphoryl)-propionsäure und 0.28 g (1.45 mMol) 4-(4-Aminophenyl)-morpholin-3-on 0.4 g (53.2%) (S)-2-(Benzyloxycarbonyl-amino)-*N*-[4-(3-oxo-morpholin-4-yl)-phenyl]-3-(dimethoxy-phosphoryl)-propionamid als farbloses Öl, ESI 505.

5.3 Die Mischung aus 0.39 g (0.78 mMol) (S)-2-(Benzy)oxycarbonylamino)-*N*-[4-(3-oxo-morpholin-4-yl)-phenyl]-3-(dimethoxy-phosphoryl)-propionamid und 0.4 g 5% Palladium/Kohle in 30 ml Methanol wird solange hydriert, bis kein Wasserstoff mehr aufgenommen wird. Anschließend wird die Reaktionsmischung abfiltriert und das Filtrat zur Trockne eingeengt. Man erhält so 0.27 g (S)-2-Amino-*N*-[4-(3-oxo-morpholin-4-yl)-phenyl]-3-(dimethoxy-phosphoryl)-propionamid als farbloses Öl, ESI 371.

5.4 Die Lösung von 0.2 g (0.54 mMol) (S)-{2-Amino-2-[4-(3-oxo-morpholin-4-yl)-phenylcarbamoyl]-ethyl}-phosphonsäuredimethylester und 0.092 g (0.54 mMol) 4-Chlorphenylisocyanat in 10 ml Dichlormethan wird 12 Stunden bei Raumtemperatur gerührt. Anschliessend wird die Methylenchloridlösung nacheinander mit je 10 ml 1N Salzsäure, gesättigter Natriumhyrogencarbonatlösung und Wasser gewaschen und über Natriumsulfat getrocknet. Nach Abziehen des Lösungsmittels wird der Rückstand mit 10 ml Diethylether versetzt und der ausgefallene weiße Niederschlag abfiltriert. Man erhält so 0.28 g (100%) (S)-2-[3-(4-Chlorphenyl)-ureido]-*N*-[4-(3-oxo-morpholin-4-yl)-phenyl]-3-(dimethoxy-phosphoryl)-propionamid ("1 D"), ESI 525.

Entsprechend erhält man
(R)-2-[3-(4-Chlorphenyl)-ureido]-*N*-[4-(3-oxo-morphotin-4-yl)-phenyl]-3-(dimethoxy-phosphoryl)-propionamid, ESI 525;
(R)-2-[3-(4-Chlorphenyl)-ureido]-*N*-[3-methyl-4-(3-oxo-morpholin-4-yl)-phenyl]-3-(dimethoxy-phosphoryl)-propionamid, ESI 539 und
(S)-2-[3-(4-Chlorphenyl)-ureido]-*N*-[3-methyl-4-(3-oxo-morpholin-4-yl)-phenyl]-3-(dimethoxy-phosphoryl)-propionamid, ESI 539.

Analog erhält man 2-[3-(4-Chlorphenyl)-ureido]-*N*-[4-(2-oxo-piperidin-1-yl)-phenyl]-3-(dimethoxy-phosphoryl)-propionamid.

Durch Hydrolyse erhält man daraus 2-[3-(4-Chlorphenyl)-ureido]-*N*-[4-(3-oxo-morpholin-4-yl)-phenyl]-3-phosphono-propionamid.

### Beispiel 6

Die Herstellung von 2-[3-(4-Chlorphenyl)-ureido]-*N*-[4-(3-oxo-morpholin-4-yl)-phenyl]-4-(methansulfoximin-yl)-butyramid erfolgt analog nachstehendem Schema:

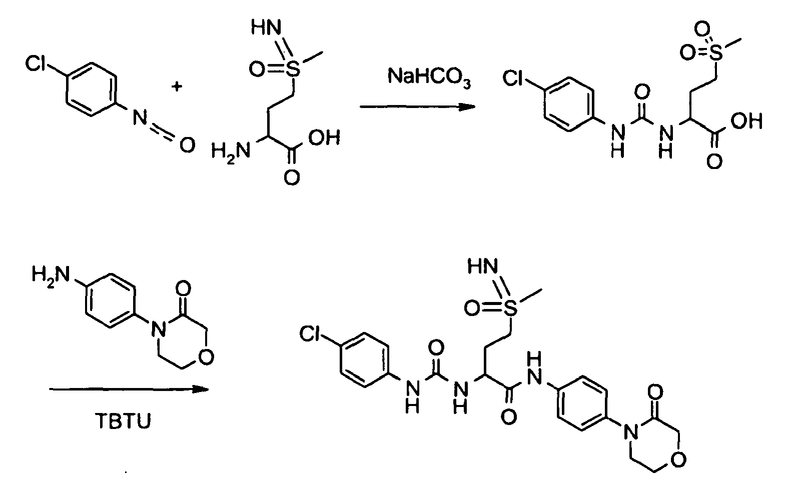

### Beispiel 7

Die Herstellung von 2-[3-(4-Chlorphenyl)-ureido]-*N*-[4-(3-oxo-morpholin-4-yl)-phenyl]-3-sulfamoyl-propionamid erfolgt analog nachstehendem Schema:

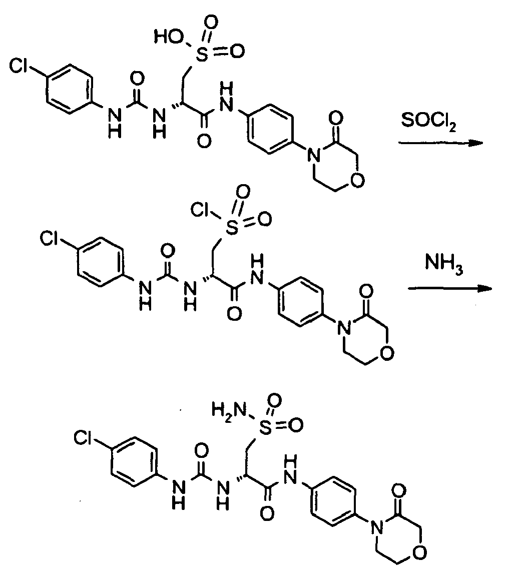

Analog erhält man 2-[3-(4-Chlorphenyl)-ureido]-*N*-[4-(2-oxo-2*H*-pyridin-1-yl)-phenyl]-3-sulfamoyl-propionamid.

### Beispiel 8

Die Herstellung von 2-[3-(4-Chlorphenyl)-ureido]-*N*-[4-(3-oxo-morpholin-4-yl)-phenyl]-3-methansulfonylamino-propionamid erfolgt analog nachstehendem Schema:

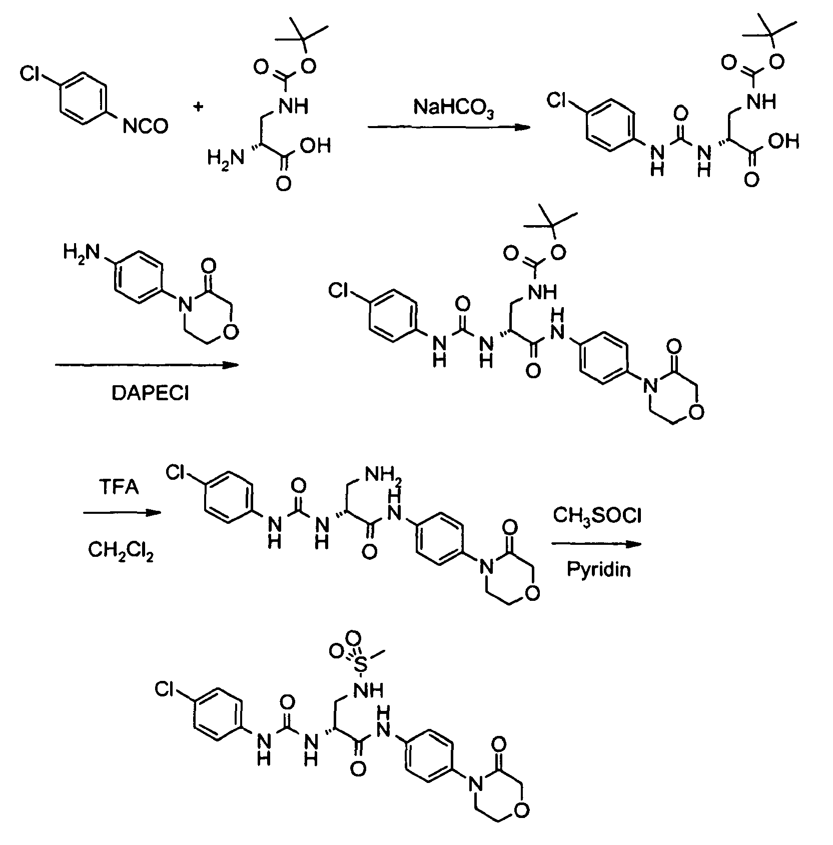

Analog erhält man die Verbindung
(R)-2-[3-(4-Chlorphenyl)-ureido]-*N*-[4-(3-oxo-morpholin-4-yl)-phenyl]-5-methansulfonylamino-valeriansäureamid, ESI 552.

### Beispiel 9

Die Herstellung von 2-[3-(4-Chlorphenyl)-ureido]-*N*-[4-(3-oxo-morpholin-4-yl)-phenyl]-3-sulfamoyloxy-propionamid 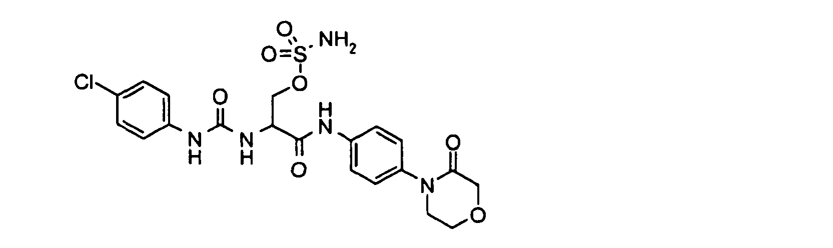 kann durch Umsetzung des Hydroxyderivates mit Chlorsulfonylisocycanat erfolgen.

Entsprechend erhält man (R)-2-[3-(4-Chlorphenyl)-ureido]-*N*-[4-(3-oxo-morpholin-4-yl)-phenyl]-3-sulfamoyloxy-propionamid, ESI 512.

Pharmakologische Daten (Affinität zu Rezeptoren)

| Verbindung Nr. | FXa-IC₅₀ [M] | TF/FVIIa-IC₅₀[M] |
|---|---|---|
| "1A" | 2.8 x 10⁻⁸ | 2.8 x 10⁻⁸ |
| "1B" | 4.2 x 10⁻⁸ | 4.3x10⁻⁸ |
| "1C" | 5.9 x 10⁻⁸ | 5.8 x 10⁻⁸ |
| "2A" | 6.4 x 10⁻⁹ | 1.2 x 10⁻⁸ |
| "1C" | 1.1 x 10⁻⁸ | 2.3 x 10⁻⁸ |
| "1D" | 8.5 x 10⁻⁸ | |

Die nachfolgenden Beispiele betreffen Arzneimittel:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ . 2 H₂O, 28,48 g Na₂HPO₄ - 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffetstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 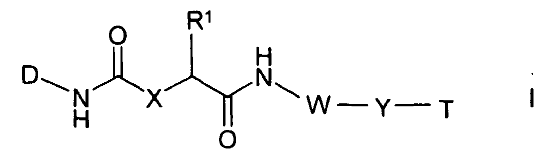 worin
D einfach durch Hal substituiertes Phenyl,
R¹ Alkyl mit 1, 2, 3, 4, 5 oder 6 C-Atomen, das einfach durch S(O)ₘR², SO₂N(R²)₂, SO₃R², S(=O)(=NR²)R², NR²SO₂R², OSO₂R², OSO₂N(R²)₂ oder PO(OR²)₂ substituiert ist,
R² H oder Alkyl mit 1, 2, 3, 4, 5 oder 6 C-Atomen,
W -(CH₂)ₙ-,
X NH oder O,
Y Ar-diyl,
T Piperidin-1-yl, 2-Oxo-piperidin-1-yl, 2-Oxo-pyrrolidin-1-yl, Pyrrolidin-1-yl, 3-Oxo-morpholin-4-yl, Morpholin-4-yl, 4- Oxo-1*H*-pyridin-1-yl, 2,6-Dioxo-piperidin1-yl, 2-Oxo- piperazin-1-yl, 2,6-Dioxo-piperazin-1-yl, 2,5-Dioxo- pyrrolidin-1-yl, 2-Oxo-1,3-oxazolidin-3-yl, 3-Oxo-2*H*- pyridazin-2-yl, 2-Caprolactam-1-yl (= 2-Oxo-azepan-1-yl), 2-Hydroxy-6-oxo-piperazin-1-yl, 2-Methoxy-6-oxo- piperazin-1-yl, 2-Aza-bicyclo[2.2.2]-octan-3-on-2-yl, 5,6- Dihydro-1*H*-pyrimidin-2-oxo-1-yl, 2-Oxo-[1,3]oxazinan-3-yl oder 4*H*-[1,4]Oxazin-4-yl, oder N(R²)₂ und falls Y = Piperidin-1,4-diyl, auch R² oder Cycloalkyl,
A unverzweigtes oder verzweigtes Alkyl mit 1-10 C-Atomen, worin eine oder zwei CH₂-Gruppen durch O- oder S-Atome und/oder durch -CH=CH-Gruppen und/oder auch 1-7 H- Atome durch F ersetzt sein können,
Ar unsubstituiertes oder ein-, zwei- oder dreifach durch Hal, A, OR², SO₂A, SO₂NH₂, COOR² oder CN substituiertes Phenyl,
Hal F, CI, Br oder I,
m 1 oder 2,
n 0, 1 oder 2 bedeuten,
sowie ihre pharmazeutisch verwendbaren Salze Solvate und Stereoisomere, einschließlich deren Mischungen in allen Verhältnissen.

2. Verbindungen nach Anspruch 1,
worin
D einfach durch Hal substituiertes Phenyl,
R¹ Alkyl mit 1, 2, 3, 4, 5 oder 6 C-Atomen, das einfach durch S(O)ₘR², SO₂N(R²)₂, SO₃R², S(=O)(=NR²)R², NR²SO₂R², OSO₂R², OSO₂N(R²)₂ oder PO(OR²)₂ substituiert ist,
R² H oder Alkyl mit 1, 2, 3, 4, 5 oder 6 C-Atomen,
W -(CH₂)ₙ-,
X NH oder O,
Y unsubstituiertes oder einfach durch A substituiertes Phenylen
T Piperidin-1-yl, 2-Oxo-piperidin-1-yl, 2-Oxo-pyrrolidin-1-yl, Pyrrolidin-1-yl, 3-Oxo-morpholin-4-yl, Morpholin-4-yl, 4- Oxo-1*H*-pyridin-1-yl, 2,6-Dioxo-piperidin1-yl, 2-Oxo- piperazin-1-yl, 2,6-Dioxo-piperazin-1-yl, 2,5-Dioxo- pyrrolidin-1-yl, 2-Oxo-1,3-oxazolidin-3-yl, 3-Oxo-2*H*- pyridazin-2-yl, 2-Caprolactam-1-yl (= 2-Oxo-azepan-1-yl), 2-Hydroxy-6-oxo-piperazin-1-yl, 2-Methoxy-6-oxo- piperazin-1-yl, 2-Aza-bicyclo[2.2.2]-octan-3-on-2-yl, 5,6- Dihydro-1*H*-pyrimidin-2-oxo-1-yl, 2-Oxo-[1,3]oxazinan-3-yl oder 4*H*-[1,4]Oxazin-4-yl, oder N(R²)₂ und falls Y = Piperidin-1,4-diyl, auch R² oder Cycloalkyl,
A unverzweigtes oder verzweigtes Alkyl mit 1-10 C-Atomen, worin eine oder zwei CH₂-Gruppen durch O- oder S-Atome und/oder durch -CH=CH-Gruppen und/oder auch 1-7H- Atome durch F ersetzt sein können,
Y unsubstituiertes oder einfach durch A substituiertes Phenylen,
Hal F, Cl, Br oder I,
m 1 oder 2,
n 0, 1 oder 2 bedeuten,
sowie ihre pharmazeutisch verwendbaren Salze Solvate und Stereoisomere, einschließlich deren Mischungen in allen Verhältnissen.

3. Verbindungen nach Anspruch 1
2-[3-(4-Chlorphenyl)-ureido]-*N*-[4-(2-oxo-2*H*-pyrazin-1-yl)-phenyl]-4-methansulfonyl-butyramid,
2-[3-(4-Chlorphenyl)-ureido]-*N*-[4-(3-oxo-morpholin-4-yl)-phenyl]-4-methansulfonyl-butyramid,
(R)-2-[3-(4-Chlorphenyl)-ureido]-*N*-[4-(3-oxo-morpholin-4-yl)-phenyl]-4-methansulfonyl-butyramid,
(R)-2-(3-(4-Chlorphenyl)-ureido]-*N*-[4-(2-oxo-2*H*-pyridin-1-yl)-phenyl]-3-methansulfonyl-propionamid,
(S)-2-(3-(4-Chlorphenyl)-ureido]-*N*-[4-(2-oxo-2*H*-pyridin-1-yl)-phenyl]-3-methansulfonyl-propionamid,
(S)-2-[*N*-(4-Chlorphenyl)-carbamoyloxy]-*N*-[4-(3-oxo-morpholin-4-yl)-phenyl]-3-methansulfonyl-propionamid,
(R)-2-[*N*-(4-Chlorphenyl)-carbamoyloxy]-*N*-[4-(3-oxo-morpholin-4-yl)-phenyl]-3-methansulfonyl-propionamid,
(R)-2-[3-(4-Chlorphenyl)-ureido]-*N*-[4-(2-oxo-2*H*-pyridin-1-yl)-phenyl]-4-methansulfonyl-butyramid,
(S)-2-[3-(4-Chlorphenyl)-ureido]-*N*-[4-(3-oxo-morpholin-4-yl)-phenyl]-3-methansulfonyl-propionamid,
2-[*N*-(4-Chlorphenyl)-carbamoyloxy]-*N*-[4-(2-oxo-2*H*-pyridin-1-yl)-phenyl]-3-methansulfonyl-propionamid,
2-[3-(4-Chlorphenyl)-ureido]-*N*-[4-(3-oxo-morpholin-4-yl)-phenyl]-3-sulfo-propionamid
2-[3-(4-Chlorphenyl)-ureido]-*N*-[4-(2-oxo-2*H*-pyrid in-1-yl)-phenyl]-3-sulfo-propionamid,
(S)-2-[3-(4-Chlorphenyl)-ureido]-*N*-[4-(3-oxo-morpholin-4-yl)-phenyl]-3-(dimethoxy-phosphoryl)-propionamid,
2-[3-(4-Chlorphenyl)-ureido]-*N*-[4-(2-oxo-piperidin-1-yl)-phenyl]-3-(dimethoxy-phosphoryl)-propionamid,
2-[3-(4-Chlorphenyl)-ureido]-*N*-[4-(3-oxo-morpholin-4-yl)-phenyl]-3-phosphono-propionamid,
2-[3-(4-Chlorphenyl)-ureido]-*N*-[4-(3-oxo-morpholin-4-yl)-phenyl]-4-(methansulfoximin-yl)-butyramid,
2-[3-(4-Chlorphenyl)-ureido]-*N*-[4-(2-oxo-2*H*-pyridin-1-yl)-phenyl]-3-sulfamoyl-propionamid,
2-[3-(4-Chlorphenyl)-ureido]-*N*-[4-(3-oxo-morpholin-4-yl)-phenyl]-3-methansulfonylamino-propionamid,
2-[3-(4-Chlorphenyl)-ureido]-*N*-[4-(3-oxo-morpholin-4-yl)-phenyl]-3-sulfamoyloxy-propionamid,
(R)-2-[3-(4-Chlorphenyl)-ureido]-*N*-[3-methyl-4-(3-oxo-morpholin-4-yl)-phenyl]-3-methansulfonyl-propionamid,
(R)-2-[3-(4-Chlorphenyl)-ureido]-*N*-[4-(2-oxo-[1,3]oxazinan-3-yl)-phenyl]-3-methansulfonyl-propionamid,
(R)-2-[3-(4-Chlorphenyl)-ureido]-*N*-[4-(3-oxo-morpholin-4-yl)-phenyl]-5-methansulfonylamino-valeriansäureamid,
(R)-2-[3-(4-Chlorphenyl)-ureido]-*N*-[3-methyl-4-(3-oxo-morpholin-4-yl)-phenyl]-4-methansulfonyl-butyramid,
(R)-2-[3-(4-Chlorphenyl)-ureido]-*N*-[4-(3-oxo-morpholin-4-yl)-phenyl]-3-sulfamoyloxy-propionamid,
(R)-2-[3-(4-Chlorphenyl)-ureido]-*N*-[4-(3-oxo-morpholin-4-yl)-phenyl]-3-(dimethoxy-phosphoryl)-propionamid,
(R)-2-[3-(4-Chlorphenyl)-ureido]-*N*-[3-methyl-4-(3-oxo-morpholin-4-yl)-phenyl]-3-(dimethoxy-phosphoryl)-propionamid,
(S)-2-[3-(4-Chlorphenyl)-ureido]-*N*-[3-methyl-4-(3-oxo-morpholin-4-yl)-phenyl]-3-(dimethoxy-phosphoryl)-propionamid,
sowie ihre pharmazeutisch verwendbaren Salze, Solvate und Stereoisomere, einschließlich deren Mischungen in allen Verhältnissen.

4. Verfahren zur Herstellung von Verbindungen der Formel I nach den Ansprüchen 1-3 sowie ihrer pharmazeutisch verwendbaren Salze, Solvate und Stereoisomere, **dadurch gekennzeichnet, daß** man
a) eine Verbindung der Formel II 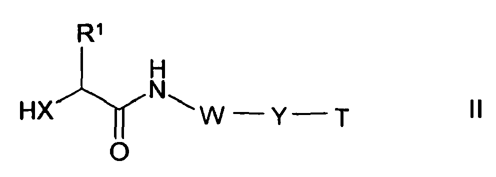 worin
R¹, T, W, X und Y die in Anspruch 1 angegebene Bedeutung haben,
mit einer Verbindung der Formel III
D-N=C=O III
worin
D die in Anspruch 1 angegebene Bedeutung hat,
umsetzt,
oder
b) eine Verbindung der Formel IV
H₂N—W—Y—T IV,
worin W, Y und T die in Anspruch 1 angegebene Bedeutung haben,
mit einer Verbindung der Formel V 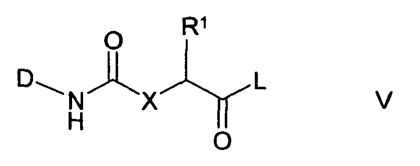 worin
L CI, Br, I oder eine freie oder reaktionsfähig funktionell abgewandelte OH-Gruppe bedeutet und
R¹, X und D die in Anspruch 1 angegebenen Bedeutungen haben,
umsetzt,
oder
c) einen Rest R¹ in einen anderen Rest R¹ umwandelt, indem man den Rest R¹ oxidiert
und/ oder eine Base oder Säure der Formel I in eines ihrer Salze umwandelt.

5. Arzneimittel, enthaltend mindestens eine Verbindung der Formel I nach einem oder mehreren der Ansprüche 1 bis 3 und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

6. Arzneimittel enthaltend mindestens eine Verbindung der Formel I gemäß einem oder mehreren der Ansprüche 1 bis 3 und/oder ihre pharmazeutisch verwendbaren Salze, Solvate und Stereoisomere, einschließlich deren Mischungen in allen Verhältnissen, und mindestens einen weiteren Arzneimittelwirkstoff.

7. Verwendung von Verbindungen gemäß einem oder mehreren der Ansprüche 1 bis 3 und/oder ihre physiologisch unbedenklichen Salze und Solvate zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen.

8. Set (Kit), bestehend aus getrennten Packungen von
(a) einer wirksamen Menge an einer Verbindung der Formel I gemäß einem oder mehreren der Ansprüche 1 bis 3 und/oder ihrer pharmazeutisch verwendbaren Salze, Solvate und Stereoisomere, einschließlich deren Mischungen in allen Verhältnissen,
und
(b) einer wirksamen Menge eines weiteren Arzneimittelswirkstoffs.

9. Verwendung von Verbindungen der Formel I gemäß einem oder mehreren der Ansprüche 1 bis 3 und/oder ihrer pharmazeutisch verwendbaren Salze, Solvate und Stereoisomere, einschließlich deren Mischungen in allen Verhältnissen,
zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen, in Kombination mit mindestens einem weiteren Arzneimittelwirkstoff.

## Claims

1. Compounds of the formula I 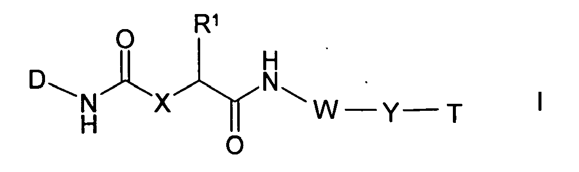 in which
D denotes phenyl which is monosubstituted by Hal,
R¹ denotes alkyl having 1, 2, 3, 4, 5 or 6 C atoms which is monosubstituted by S(O)ₘR², SO₂N(R²)₂, SO₃R², S(=O)(=NR²)R², NR²SO₂R², OSO₂R², OSO₂N(R²)₂ or PO(OR²)₂,
R² denotes H or alkyl having 1, 2, 3, 4, 5 or 6 C atoms,
W denotes -(CH₂)ₙ-,
X denotes NH or O,
Y denotes Ar-diyl,
T denotes piperidin-1-yl, 2-oxopiperidin-1-yl, 2-oxopyrrolidin- 1-yl, pyrrolidin-1-yl, 3-oxomorpholin-4-yl, morpholin-4-yl, 4-oxo-1H-pyridin-1-yl, 2,6-dioxopiperidin-1-yl, 2-oxopipera- zin-1-yl, 2,6-dioxopiperazin-1-yl, 2,5-dioxopyrrolidin-1-yl, 2-oxo-1,3-oxazolidin-3-yl, 3-oxo-2*H*-pyridazin-2-yl, 2-capro- lactam-1-yl (= 2-oxoazepan-1-yl), 2-hydroxy-6-oxopipera- zin-1-yl, 2-methoxy-6-oxopiperazin-1-yl, 2-azabicyclo- [2.2.2]octan-3-on-2-yl, 5,6-dihydro-1*H*-pyrimidin-2-oxo-1-yl, 2-oxo-1,3-oxazinan-3-yl or 4*H*-1,4-oxazin-4-yl, or N(R²)₂ and, if Y = piperidine-1,4-diyl, also R² or cycloalkyl,
A denotes unbranched or branched alkyl having 1-10 C atoms, in which one or two CH₂ groups may be replaced by O or S atoms and/or by -CH=CH- groups and/or also 1-7 H atoms may be replaced by F,
Ar denotes phenyl which is unsubstituted or mono-, di- or trisubstituted by Hal, A, OR²,SO₂A, SO₂NH₂, COOR² or CN,
Hal denotes F, CI, Br or I,
m denotes 1 or 2,
n denotes 0, 1 or 2,
and pharmaceutically usable salts, solvates and stereoisomers thereof, including mixtures thereof in all ratios.

2. Compounds according to Claim 1
in which
D denotes phenyl which is monosubstituted by Hal,
R¹ denotes alkyl having 1, 2, 3, 4, 5 or 6 C atoms which is monosubstituted by S(O)ₘR², SO₂N(R²)₂, SO₃R², S(=O)(=NR²)R₂, NR²SO²R², OSO₂R², OSO₂N(R²)₂ or PO(OR²)₂,
R² denotes H or alkyl having 1, 2, 3, 4, 5 or 6 C atoms,
W denotes -(CH₂)ₙ-,
X denotes NH or O,
Y denotes phenylene which is unsubstituted or monosubsti- tuted by A,
T denotes piperidin-1-yl, 2-oxopiperidin-1-yl, 2-oxopyrrolidin- 1-yl, pyrrolidin-1-yl, 3-oxomorpholin-4-yl, morpholin-4-yl, 4-oxo-1*H*-pyridin-1-yl, 2,6-dioxopiperidin-1-yl, 2-oxopipera- zin-1-yl, 2,6-dioxopiperazin-1-yl, 2,5-dioxopyrrolidin-1-yl, 2-oxo-1,3-oxazolidin-3-yl, 3-oxo-2*H*-pyridazin-2-yl, 2-capro- lactam-1-yl (= 2-oxoazepan-1-yl), 2-hydroxy-6-oxopipera- zin-1-yl, 2-methoxy-6-oxopiperazin-1-yl, 2-azabicyclo- [2.2.2]octan-3-on-2-yl, 5,6-dihydro-1*H*-pyrimidin-2-oxo-1-yl, 2-oxo-1,3-oxazinan-3-yl or 4*H*-1,4-oxazin-4-yl, or N(R²)₂ and, if Y = piperidine-1,4-diyl, also R² or cycloalkyl,
A denotes unbranched or branched alkyl having 1-10 C atoms, in which one or two CH₂ groups may be replaced by O or S atoms and/or by -CH=CH- groups and/or also 1-7 H atoms may be replaced by F,
Y denotes phenylene which is unsubstituted or monosubsti- tuted by A,
Hal denotes F, CI, Br or I,
m denotes 1 or 2,
n denotes 0, 1 or 2,
and pharmaceutically usable salts, solvates and stereoisomers thereof, including mixtures thereof in all ratios.

3. Compounds according to Claim 1
2-[3-(4-chlorophenyl)ureido]-*N*-[4-(2-oxo-2*H*-pyrazin-1-yl)-phenyl]-4-methanesulfonylbutyramide,
2-[3-(4-chlorophenyl)ureido]-*N*-[4-(3-oxomorpholin-4-yl)phenyl]-4-methanesulfonylbutyramide,
(R)-2-[3-(4-chlorophenyl)ureido]-*N*-[4-(3-oxomorpholin-4-yl)-phenyl]-4-methanesulfonylbutyramide,
(R)-2-[3-(4-chlorophenyl)ureido]-*N*-[4-(2-oxo-2*H*-pyridin-1-yl)-phenyl]-3-methanesulfonylpropionamide,
(S)-2-[3-(4-chlorophenyl)ureido]-*N*-[4-(2-oxo-2*H*-pyridin-1-yl)-phenyl]-3-methanesulfonylpropionamide,
(S)-2-[*N*-(4-chlorophenyl)carbamoyloxy]-*N*-[4-(3-oxomorpholin-4-yl)phenyl]-3-methanesulfonylpropionamide,
(R)-2-[*N*-(4-chlorophenyl)carbamoyloxy]-*N*-[4-(3-oxomorpholin-4-yl)phenyl]-3-methanesulfonylpropionamide,
(R)-2-[3-(4-chlorophenyl)ureido]-*N*-[4-(2-oxo-2*H*-pyridin-1-yl)-phenyl]-4-methanesulfonylbutyramide,
(S)-2-[3-(4-chlorophenyl)ureido]-*N*-[4-(3-oxomorpholin-4-yl)-phenyl]-3-methanesulfonylpropionamide,
2-[N-(4-chlorophenyl)carbamoyloxy]-*N*-[4-(2-oxo-2*H*-pyridin-1-yl)phenyl]-3-methanesulfonylpropionamide,
2-[3-(4-chlorophenyl)ureido]-*N*-[4-(3-oxomorpholin-4-yl)phenyl]-3-sulfopropionamide,
2-[3-(4-chlorophenyl)ureido]-*N*-[4-(2-oxo-2*H*-pyridin-1-yl)phenyl]-3-sulfopropionamide,
(S)-2-[3-(4-chlorophenyl)ureido]-*N*-[4-(3-oxomorpholin-4-yl)-phenyl]-3-(dimethoxyphosphoryl)propionamide,
2-[3-(4-chlorophenyl)ureido]-*N*-[4-(2-oxopiperidin-1-yl)phenyl]-3-(dimethoxyphosphoryl)propionamide,
2-[3-(4-chlorophenyl)ureido]-*N*-[4-(3-oxomorpholin-4-yl)phenyl]-3-phosphonopropionamide,
2-(3-(4-chlorophenyl)ureido]-*N*-[4-(3-oxomorpholin-4-yl)phenyl]-4-(methanesulfoximinyl)butyramide,
2-[3-(4-chlorophenyl)ureido]-*N*-[4-(2-oxo-2*H*-pyridin-1-yl)phenyl]-3-sulfamoylpropionamide,
2-[3-(4-chlorophenyl)ureido]-*N*-[4-(3-oxomorpholin-4-yl)phenyl]-3-methanesulfonylaminopropionamide,
2-[3-(4-chlorophenyl)ureido]-*N-*(4-(3-oxomorpholin-4-yl)phenyl]-3-sulfamoyloxypropionamide,
(R)-2-[3-(4-chlorophenyl)ureido]-*N*-[3-methyl-4-(3-oxomorpholin-4-yl)phenyl]-3-methanesulfonylpropionamide,
(R)-2-[3-(4-chlorophenyl)ureido]-*N*-[4-(2-oxo-1,3-oxazinan-3-yl)-phenyl]-3-methanesulfonylpropionamide,
(R)-2-[3-(4-chlorophenyl)ureido]-*N-*[3-methyl-4-(3-oxomorpholin-4-yl)phenyl]-5-methanesulfonylvaleramide,
(R)-2-[3-(4-chlorophenyl)ureido]-*N-*[3-methyl-4-(3-oxomorpholin-4-yl)phenyl]-4-methanesulfonylbutyramide,
(R)-2-[3-(4-chlorophenyl)ureido]-*N-*[4-(3-oxomorpholin-4-yl)-phenyl]-3-sulfamoyloxypropionamide,
(R)-2-[3-(4-chlorophenyl)ureido]-*N-*[4-(3-oxomorpholin-4-yl)-phenyl]-3-(dimethoxyphosphoryl)propionamide,
(R)-2-[3-(4-chlorophenyl)ureido]-*N-*[3-methyl-4-(3-oxomorpholin-4-yl)phenyl]-3-(dimethoxyphosphoryl)propionamide,
(S)-2-[3-(4-chlorophenyl)ureido]-*N*-[3-methyl-4-(3-oxomorpholin-4-yl)phenyl]-3-(dimethoxyphosphoryl)propionamide,
and pharmaceutically usable salts, solvates and stereoisomers thereof, including mixtures thereof in all ratios.

4. Process for the preparation of compounds of the formula I according to Claims 1-3 and pharmaceutically usable salts, solvates and stereoisomers thereof, **characterised in that**
a) a compound of the formula II 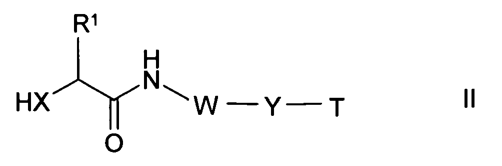 in which
R¹, T, W, X and Y have the meaning indicated in Claim 1,
is reacted with a compound of the formula III
D-N=C=O III
in which
D has the meaning indicated in Claim 1,
or
b) a compound of the formula IV
H₂N-W-Y-T IV,
in which W, Y and T have the meaning indicated in Claim 1,
is reacted with a compound of the formula V 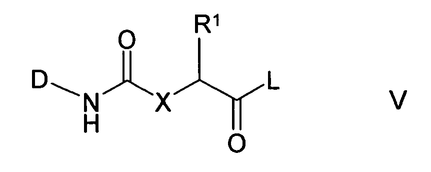 in which
L denotes Cl, Br, I or a free or reactively functionally modified OH group and
R¹, X and D have the meanings indicated in Claim 1,
or
c) a radical R¹ is converted into another radical R¹ by oxidising the radical R¹
and/or a base or acid of the formula I is converted into one of its salts.

5. Medicaments comprising at least one compound of the formula I according to one or more of Claims 1 to 3 and/or pharmaceutically usable salts, solvates and stereoisomers thereof, including mixtures thereof in all ratios, and optionally excipients and/or adjuvants.

6. Medicaments comprising at least one compound of the formula I according to one or more of Claims 1 to 3 and/or pharmaceutically usable salts, solvates and stereoisomers thereof, including mixtures thereof in all ratios, and at least one further medicament active ingredient.

7. Use of compounds according to one or more of Claims 1 to 3 and/or physiologically acceptable salts and solvates thereof for the preparation of a medicament for the treatment of thromboses, myocardial infarction, arteriosclerosis, inflammation, apoplexy, angina pectoris, restenosis after angioplasty, claudicatio intermittens, migraine, tumours, tumour diseases and/or tumour metastases.

8. Set (kit) consisting of separate packs of
(a) an effective amount of a compound of the formula I according to one or more of Claims 1 to 3 and/or pharmaceutically usable salts, solvates and stereoisomers thereof, including mixtures thereof in all ratios,
and
(b) an effective amount of a further medicament active ingredient.

9. Use of compounds of the formula I according to one or more of Claims 1 to 3 and/or pharmaceutically usable salts, solvates and stereoisomers thereof, including mixtures thereof in all ratios, for the preparation of a medicament for the treatment of thromboses, myocardial infarction, arteriosclerosis, inflammation, apoplexy, angina pectoris, restenosis after angioplasty, claudicatio intermittens, migraine, tumours, tumour diseases and/or tumour metastases, in combination with at least one further medicament active ingredient.

## Revendications

1. Composés de formule I 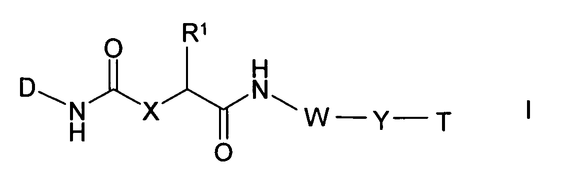 dans laquelle
D désigne phényle qui est monosubstitué par Hal,
R¹ désigne alkyle ayant 1, 2, 3, 4, 5 ou 6 atomes de C qui est monosubstitué par S(O)ₘR², SO₂N(R²)₂, SO₃R², S(=O)(=NR²)R², NR²SO₂R², OSO₂R², OSO₂N(R²)₂ ou PO(OR²)₂,
R² désigne H ou alkyle ayant 1, 2, 3, 4, 5 ou 6 atomes de C,
W désigne -(CH₂)ₙ-,
X désigne NH ou O,
Y désigne Ar-diyle,
T désigne pipéridin-1-yle, 2-oxopipéridin-1-yle, 2-oxopyrroli- din-1-yle, pyrrolidin-1-yle, 3-oxomorpholin-4-yle, morpholin- 4-yle, 4-oxo-1*H-*pyridin-1-yle, 2,6-dioxopipéridin-1-yle, 2- oxopipérazin-1-yle, 2,6-dioxopipérazin-1-yle, 2,5-dioxo- pyrrolidin-1-yle, 2-oxo-1,3-oxazolidin-3-yle, 3-oxo-2*H-*pyri- dazin-2-yle, 2-caprolactam-1-yle (= 2-oxoazépan-1-yle), 2- hydroxy-6-oxopipérazin-1-yle, 2-méthoxy-6-oxopipérazin-1- yle, 2-azabicyclo[2.2.2]octan-3-on-2-yle, 5,6-dihydro-1*H-* pyrimidin-2-oxo-1-yle, 2-oxo-1,3-oxazinan-3-yle ou 4*H-*1,4- oxazin-4-yle, ou N(R²)₂ et, si Y = pipéridine-1,4-diyle, de même R² ou cycloalkyle,
A désigne alkyle non ramifié ou ramifié ayant 1-10 atomes de C, où un ou deux groupements CH₂ peuvent être rempla- cés par des atomes de O ou S et/ou par des groupements -CH=CH- et/ou également 1-7 atomes de H peuvent être remplacés par F,
Ar désigne phényle qui est non substitué ou mono-, di- ou tri- substitué par Hal, A, OR², SO₂A, SO₂NH₂, COOR² ou CN,
Hal désigne F, CI, Br ou I,
m désigne 1 ou 2,
n désigne 0, 1 ou 2,
et les sels, solvats et stéréoisomères pharmaceutiquement utilisables de ceux-ci, y compris des mélanges de ceux-ci en toutes proportions.

2. Composés selon la revendication 1
dans lesquels
D désigne phényle qui est monosubstitué par Hal,
R¹ désigne alkyle ayant 1, 2, 3, 4, 5 ou 6 atomes de C qui est monosubstitué par S(O)ₘR², SO₂N(R²)₂, SO₃R², S(=O)(=NR²)R², NR²SO₂R², OSO₂R², OSO₂N(R²)₂ ou PO(OR²)₂,
R² désigne H ou alkyle ayant 1, 2, 3, 4, 5 ou 6 atomes de C,
W désigne -(CH₂)ₙ-,
X désigne NH ou O,
Y désigne phénylène qui est non substitué ou monosubstitué par A,
T désigne pipéridin-1-yle, 2-oxopipéridin-1-yle, 2-oxopyrroli- din-1-yle, pyrrolidin-1-yle, 3-oxomorpholin-4-yle, morpholin- 4-yle, 4-oxo-1*H-*pyridin-1-yle, 2,6-dioxopipéridin-1-yle, 2- oxopipérazin-1-yle, 2,6-dioxopipérazin-1-yle, 2,5-dioxo- pyrrolidin-1-yle, 2-oxo-1,3-oxazolidin-3-yle, 3-oxo-2*H-*pyri- dazin-2-yle, 2-caprolactam-1-yle (= 2-oxoazépan-1-yle), 2- hydroxy-6-oxopipérazin-1-yle, 2-méthoxy-6-oxopipérazin-1- yle, 2-azabicyclo[2.2.2]octan-3-on-2-yle, 5,6-dihydro-1*H-* pyrimidin-2-oxo-1-yle, 2-oxo-1,3-oxazinan-3-yle ou 4*H-*1,4- oxazin-4-yle, ou N(R²)₂ et, si Y = pipéridine-1,4-diyle, de même R² ou cycloalkyle,
A désigne alkyle non ramifié ou ramifié ayant 1-10 atomes de C, où un ou deux groupements CH₂ peuvent être rem- placés par des atomes de O ou S et/ou par des groupe- ments -CH=CH- et/ou également 1-7 atomes de H peuvent être remplacés par F,
Y désigne phénylène qui est non substitué ou monosubstitué par A,
Hal désigne F, CI, Br ou I,
m désigne 1 ou 2,
n désigne 0, 1 ou 2,
et les sels, solvats et stéréoisomères pharmaceutiquement utilisables de ceux-ci, y compris des mélanges de ceux-ci en toutes proportions.

3. Composés selon la revendication 1
le 2-[3-(4-chlorophényl)uréido]-*N-*[4-(2-oxo-2*H-*pyrazin-1-yl)-phényl]-4-méthanesulfonylbutyramide,
le 2-[3-(4-chlorophényl)uréido]-*N-*[4-(3-oxomorpholin-4-yl)-phényl]-4-méthanesulfonylbutyramide,
le (R)-2-[3-(4-chlorophényl)uréido]-*N-*[4-(3-oxomorpholin-4-yl)-phényl]-4-méthanesulfonylbutyramide,
le (R)-2-[3-(4-chlorophényl)uréido]-*N-*[4-(2-oxo-2*H-*pyridin-1-yl)-phényl]-3-méthanesulfonylpropionamide,
le (S)-2-[3-(4-chlorophényl)uréido]-*N-*[4-(2-oxo-2*H-*pyridin-1-yl)-phényl]-3-méthanesulfonylpropionamide,
le (S)-2-[*N-*(4-chlorophényl)carbamoyloxy]-*N-*[4-(3-oxomorpholin-4-yl)phényl]-3-méthanesulfonylpropionamide,
le (R)-2-[*N-*(4-chlorophényl)carbamoyloxy]-*N-*[4-(3-oxomorpholin-4-yl)phényl]-3-méthanesulfonylpropionamide,
le (R)-2-[3-(4-chlorophényl)uréido]-*N-*[4-(2-oxo-2*H-*pyridin-1-yl)-phényl]-4-méthanesulfonylbutyramide,
le (S)-2-[3-(4-chlorophényl)uréido]-*N-*[4-(3-oxomorpholin-4-yl)-phényl]-3-méthanesulfonylpropionamide,
le 2-[*N-*(4-chlorophényl)carbamoyloxy]-*N-*[4-(2-oxo-2*H-*pyridin-1-yl)phényl]-3-méthanesulfonylpropionamide,
le 2-[3-(4-chlorophényl)uréido]-*N-*[4-(3-oxomorpholin-4-yl)-phényl]-3-sulfopropionamide,
le 2-[3-(4-chlorophényl)uréido]-*N-*[4-(2-oxo-2*H-*pyridin-1-yl)-phényl]-3-sulfopropionamide,
le (S)-2-[3-(4-chlorophényl)uréido]-*N-*[4-(3-oxomorpholin-4-yl)-phényl]-3-(diméthoxyphosphoryl)propionamide,
le 2-[3-(4-chlorophényl)uréido]-*N-*[4-(2-oxopipéridin-1-yl)phényl]-3-(diméthoxyphosphoryl)propionamide,
le 2-[3-(4-chlorophényl)uréido]-*N-*[4-(3-oxomorpholin-4-yl)-phényl]-3-phosphonopropionamide,
le 2-[3-(4-chlorophényl)uréido]-*N-*[4-(3-oxomorpholin-4-yl)-phényl]-4-(méthanesulfoximinyl)butyramide,
le 2-[3-(4-chlorophényl)uréido]-*N-*[4-(2-oxo-2*H-*pyridin-1-yl)-phényl]-3-sulfamoylpropionamide,
le 2-[3-(4-chlorophényl)uréido]-*N-*[4-(3-oxomorpholin-4-yl)-phényl]-3-méthanesulfonylaminopropionamide,
le 2-[3-(4-chlorophényl)uréido]-*N-*[4-(3-oxomorpholin-4-yl)-phényl]-3-sulfamoyloxypropionamide,
le (R)-2-[3-(4-chlorophényl)uréido]-*N-*[3-méthyl-4-(3-oxomorpholin-4-yl)phényl]-3-méthanesulfonylpropionamide,
le (R)-2-[3-(4-chlorophényl)uréido]-*N-*[4-(2-oxo-1,3-oxazinan-3-yl)phényl]-3-méthanesulfonylpropionamide,
le (R)-2-[3-(4-chlorophényl)uréido]-*N-*[3-méthyl-4-(3-oxomorpholin-4-yl)phényl]-5-méthanesulfonylvaléramide,
le (R)-2-[3-(4-chlorophényl)uréido]-*N-*[3-méthyl-4-(3-oxomorpholin-4-yl)phényl]-4-méthanesulfonylbutyramide,
le (R)-2-[3-(4-chlorophényl)uréido]-*N-*[4-(3-oxomorpholin-4-yl)-phényl]-3-sulfamoyloxypropionamide,
le (R)-2-[3-(4-chlorophényl)uréido]-*N-*[4-(3-oxomorpholin-4-yl)-phényl]-3-(diméthoxyphosphoryl)propionamide,
le (R)-2-[3-(4-chlorophényl)uréido]-*N-*[3-méthyl-4-(3-oxomorpholin-4-yl)phényl]-3-(diméthoxyphosphoryl)propionamide,
le (S)-2-[3-(4-chlorophényl)uréido]-*N-*[3-méthyl-4-(3-oxomorpholin-4-yl)phényl]-3-(diméthoxyphosphoryl)propionamide,
et les sels, solvats et stéréoisomères pharmaceutiquement utilisables de ceux-ci, y compris des mélanges de ceux-ci en toutes proportions.

4. Procédé de préparation de composés de formule I selon les revendications 1-3 et de sels, solvats et stéréoisomères pharmaceutiquement utilisables de ceux-ci, **caractérisé en ce que**
a) un composé de formule II 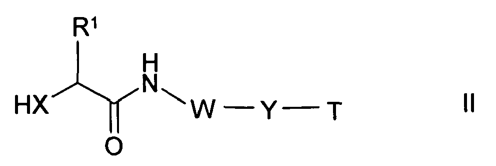 dans laquelle
R¹, T, W, X et Y revêtent la signification indiquée selon la revendication 1,
est réagi avec un composé de formule III
D-N=C=O III
dans laquelle
D revêt la signification indiquée selon la revendication 1,
ou
b) un composé de formule IV
H₂N—vV—Y—T IV,
dans laquelle W, Y et T revêtent la signification indiquée selon la revendication 1,
est réagi avec un composé de formule V 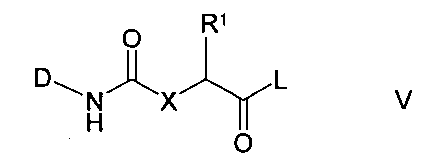 dans laquelle
L désigne CI, Br, I ou un groupement OH libre ou modifié de manière fonctionnelle et réactive et
R¹, X et D revêtent la signification indiquée selon la revendication 1,
ou
c) un radical R¹ est converti en un autre radical R¹ par l'oxydation du radical R¹
et/ou une base ou un acide de formule I est converti(e) en l'un de ses sels.

5. Médicaments comprenant au moins un composé de formule I selon l'une ou plusieurs des revendications 1 à 3 et/ou des sels, solvats et stéréoisomères pharmaceutiquement utilisables de celui-ci, y compris des mélanges de ceux-ci en toutes proportions, et éventuellement des excipients et/ou des adjuvants.

6. Médicaments comprenant au moins un composé de formule I selon l'une ou plusieurs des revendications 1 à 3 et/ou des sels, solvats et stéréoisomères pharmaceutiquement utilisables de celui-ci, y compris des mélanges de ceux-ci en toutes proportions, et au moins un autre ingrédient actif médicamenteux.

7. Utilisation de composés selon l'une ou plusieurs des revendications 1 à 3 et/ou de sels et solvats physiologiquement acceptables de ceux-ci pour la préparation d'un médicament destiné au traitement des thromboses, de l'infarctus du myocarde, de l'artériosclérose, des inflammations, de l'apoplexie, de l'angine de poitrine, de la resténose post-angioplastie, de la boiterie intermittente, de la migraine, des tumeurs, des maladies tumorales et/ou des métastases tumorales.

8. Ensemble (kit) constitué de conditionnements séparés
(a) d'une quantité efficace d'un composé de formule I selon l'une ou plusieurs des revendications 1 à 3 et/ou de sels, solvats et stéréoisomères pharmaceutiquement utilisables de celui-ci, y compris des mélanges de ceux-ci en toutes proportions,
et
(b) d'une quantité efficace d'un autre ingrédient actif médicamenteux.

9. Utilisation de composés de formule I selon l'une ou plusieurs des revendications 1 à 3 et/ou de sels, solvats et stéréoisomères pharmaceutiquement utilisables de ceux-ci, y compris des mélanges de ceux-ci en toutes proportions,
pour la préparation d'un médicament destiné au traitement des thromboses, de l'infarctus du myocarde, de l'artériosclérose, des inflammations, de l'apoplexie, de l'angine de poitrine, de la resténose post-angioplastie, de la boiterie intermittente, de la migraine, des tumeurs, des maladies tumorales et/ou des métastases tumorales, en association avec au moins au autre ingrédient actif médicamenteux.
